# EUROPÄISCHE PATENTSCHRIFT

(11) **EP 0 444 266 B1**
(45) Veröffentlichungstag und Bekanntmachung des Hinweises auf die Patenterteilung: **15.02.1995**
(21) Anmeldenummer: 90123933.5
(22) Anmeldetag: 12.12.1990
(51) Int. Cl.: C07D 239/50

(54) **Verfahren zur Herstellung von 2,4,5-Triamino-6-hydroxypyrimidin durch katalytische Hydrierung von 2,4-Diamino-6-hydroxy-5-nitrosopyrimidin**
Process for the preparation of 2,4,5-triamino-6-hydroxypyrimidine by catalytic hydrogenation of 2,4-diamino-6-hydroxy-5-nitrosopyrimidine
Procédé pour la préparation de 2,4,5-triamino-6-hydroxypyrimidine par hydrogénation catalytique de la 2,4-diamino-6-hydroxy-5-nitrosopyrimidine

(30) Priorität: 02.03.1990 DE 4006538
(43) Veröffentlichungstag der Anmeldung: 04.09.1991
(73) Patentinhaber: HÜLS AKTIENGESELLSCHAFT, 45764 Marl (DE)
(72) Erfinder: Hunds, Artur, Dr., W-5300 Bonn (DE); Rogler, Walter, W-5300 Bonn (DE)

(56) Entgegenhaltungen:
- EP-A- 0 267 594
- US-A- 2 447 523
- PATENT ABSTRACTS OF JAPAN vol. 7, no. 91 (C-162)(1236) 15 April 1983, & JP-A-58 18367 (KOJIN KK) 02 Februar 1983,

## Beschreibung

Die Erfindung betrifft ein verbessertes Verfahren zur katalytischen Hydrierung von 2,4-Diamino-6-hydroxy-5-nitrosopyrimidin (DAHNP), das dabei in 2,4,5-Triamino-6-hydroxypyrimidin (TAHP) überführt wird.

Die katalytische Hydrierung von DAHNP ist bekannt, z.B. aus US-PS 2,447,523 und DE-PS 36 38 635. Nach der US-PS wird eine 0,4 bis 0,5 molare Suspension von DAHNP in 0,1 bis 1,0 normaler Natronlauge (entsprechend 0,2 bis 2,5 mol NaOH pro mol DAHNP) unter Verwendung von Pd, PtO₂ oder Raney-Nickel als Katalysator bei 1,4 bis 2,3 bar hydriert. Diese Verfahrensweise besitzt mehrere Nachteile. Erstens ist die Konzentration des DAHNP auf 0,4 bis 0,5 mol/l begrenzt, weil sich bei höheren Konzentrationen das Reaktionsgemisch sehr schnell verdickt und nicht mehr wirksam durchmischt werden kann, so daß die Aufnahme des Wasserstoffs sich stark verlangsamt. Zweitens ist der Katalysatorbedarf mit 50 g 5 Gew.%igem Pd/C (entspr. 2,5 g metallisches Pd) 4 g PtO₂ oder 150 g Raney-Nickel pro mol DAHNP hoch. Beide Nachteile werden durch die DE-PS 36 38 635 vermieden. Hier wird DAHNP in wäßriger Lösung bei 20 bis 80 °C in einer Konzentration 0,5 bis 3,0 mol/l unter Verwendung von 0,02 bis 0,2 g Pd-Metall (1 bis 10 Gew- % auf Kohle) pro mol DAHNP unter einen Wasserstoffdruck von 1,4 bis 21 bar hydriert, wobei 0,8 bis 1,5 mol Natronlauge pro mol DAHNP während der Hydrierung zudosiert werden.

Trotz dieser bereits erheblichen Vorteile hat aber auch dieses Verfahren noch den Nachteil, daß größtenteils im stark alkalischen Bereich gearbeitet wird. Bereits bei 0,2 mol Alkali pro mol DAHNP - der kleinsten Alkalimenge nach der US-PS - stellt sich jedoch bereits ein pH von 12 und - nach der DE-PS - mit NH₃ pH 10 und mehr ein. Versuche, das DAHNP in wäßriger saurer Lösung zu hydrieren, waren jedoch nach US-PS 2,447,523 Spalte 1 Absatz 3 erfolglos.

Wir haben nun gefunden, daß die Hydrierung im alkalischen Bereich den großen Nachteil hat, daß die wertvollen katalytisch aktiven Edelmetalle während der Hydrierung in beträchtlichen Mengen (bis über 50 %) aufgelöst werden. Sie werden dann bei der Abfiltration des Katalysators nicht zurückgehalten und gelangen ins Produktionsabwasser und in das Produkt TAHP-Sulfat und können daraus mit vertretbarem Aufwand nicht wiedergewonnen werden. Wegen des hohen Preises der verwendeten Edelmetalle beeinträchtigen bereits geringe Verluste die Wirtschaftlichkeit des Prozesses. Weiterhin ist die Kontaminierung des TAHP-Sulfats, das zu pharmazeutisch wertvollen Substanzen wie z.B. Guanin und Folsäure weiterverarbeitet wird, sehr bedenklich. Auch das ökologische Verhalten der genannten Metalle in den Vorflutern ist weitgehend unbekannt.

Es war daher die Aufgabe der vorliegenden Erfindung, die Verluste von Edelmetall bei der katalytischen Hydrierung des DAHNP zu vermeiden.

Diese Aufgabe wird durch das erfindungsgemäße Verfahren gelöst, indem die Hydrierung des DAHNP bei erhöhtem Druck und erhöhter Temperatur in einem wäßrigen, gegebenenfalls noch organischen Lösemittel und/oder inerte anorganische Salze enthaltenden Medium in Gegenwart eines Edelmetallkatalysators erfolgt, wobei die Hydrierung im sauren, neutralen oder schwach alkalischen Bereich unterhalb pH 9 erfolgt, und wobei vorzugsweise vor und während der Hydrierung die Zugabe alkalischer Mittel unterbleibt und nach der Hydrierung die zur Lösung von TAHP notwendige Menge alkalischer Mittel zugegeben und die entstehende Lösung vom Katalysator getrennt wird. Vorzugsweise wird die Reaktion im pH-Bereich von 2,5 bis 9,0, sehr bevorzugt von 3 bis 8,5, durchgeführt. Das Verfahren schließt somit auch die Hydrierung unter sauren Bedingungen ein. Es wurde gefunden, daß bei erhöhter Temperatur und erhöhtem Druck selbst bei einem pH-Wert von 2 die Hydrierung problemlos ablief. Derart niedrige pH-Werte sind allerdings nur sinnvoll, sofern nicht Korrosion des Materials der Hydrierapparatur auftritt.

Gegenstand der Erfindung ist daher das Verfahren nach dem Patentanspruch und den Unteransprüchen.

Zur Durchführung des erfindungsgemäßen Verfahrens wird das in Wasser suspendierte DAHNP oder die bei der Herstellung des DAHNP nach einem bekannten Verfahren erhaltene, gegebenenfalls noch Prozeßsalze und/oder organische Lösemittel enthaltende wäßrige DAHNP-Suspension mit einem Hydrierkatalysator versetzt und bei einem Druck im Bereich von 3 bis 150 bar und Temperaturen von 50 bis 150 °C mit Wasserstoff umgesetzt.

Im Gegensatz zu den bisher bekannten Verfahren erfolgt die Zugabe der zum Lösen des bei der Hydrierung entstandenen 2,4,5-Triamino-6-hydroxypyrimidins (TAHP) und Abtrennen dieser Lösung vom Katalysator erforderliche Base erst nach beendeter Reaktion. Durch diese erfindungsgemäße Verfahrensweise wird die Auflösung der Edelmetalle unterdrückt.

Die Konzentration an DAHNP kann 0,4 bis 3 mol/l betragen, wobei mit isoliertem DAHNP eine Konzentration von 1,5 bis 2,5 mol/l bevorzugt wird, während bei Verwendung der bei der Herstellung anfallenden salzhaltigen DAHNP-Suspension 0,6 bis 1 mol/l bevorzugt werden.

Der Wasserstoffdruck ist nicht kritisch, zur Erzielung höherer Reaktionsgeschwindigkeiten werden 10 bis 60 bar bevorzugt. Der bevorzugte Temperaturbereich liegt zwischen 70 und 120 °C. Bei tieferen Temperaturen ist die Reaktionsgeschwindigkeit deutlich geringer und bei Temperaturen von mehr als 120°C weisen die erhaltenen Produkte oft eine dunklere Farbe auf.

Als Hydrierkatalysatoren werden Edelmetalle wie Ru, Pd oder Pt in fein verteilter Form, bevorzugt auf Trägermaterialien, insbesondere Aktivkohlen, die 1 bis 10 Gew.-%, bevorzugt 4 bis 6 Gew.-% Palladium oder Platin oder ausreichende Mengen der Oxide dieser Metalle enthalten, eingesetzt. Pro mol DAHNP werden sehr bevorzugt 0,2 bis 0,02 g Pd-Metall bzw. 0,4 bis 0,04 g Pt-Metall eingesetzt.

Die Reaktion kann in allen herkömmlichen Druckreaktoren mit Rühr- oder Mischeinrichtungen durchgeführt werden, besonders bevorzugt sind jedoch Treibstrahl-Schlaufenreaktoren. Erst nach beendeter Reaktion wird 0,9 bis 1,2 mol Alkalihydroxid zugegeben, bevorzugt 1,1 mol pro mol DAHNP einer wäßrigen Lösung von Natrium- oder Kaliumhydroxyd, um das ausgefallene TAHP aufzulösen. Dann wird in bekannter Weise vom Katalysator abfiltriert und das oxidationsempfindliche TAHP beispielsweise durch Einlaufenlassen des Filtrats in wäßrige Schwefelsäure als stabiles Sulfat gefällt.

Der Vorteil des beschriebenen Verfahrens liegt in stark verringerten Verlusten der Edelmetall-Katalysatoren. Bei den hohen Preisen der katalytisch aktiven Edelmetalle wird dadurch die Wirtschaftlichkeit des Verfahrens sehr verbessert und eine Kontaminierung des Produktes TAHP-Sulfat und des Produktionsabwassers verhindert. Dadurch ergibt sich als weiterer Vorteil, daß der eingesetzte Katalysator, besonders nach Zusatz einer geringen Menge frischen Katalysators, für weitere Ansätze genutzt werden kann, wodurch die Wirtschaftlichkeit des Prozesses noch weiter verbessert wird. Frischer Katalysator kann in Mengen von 0 bis 50 %, vorzugsweise 5 bis 25 %, zu gebrauchtem Katalysator zugesetzt werden.

Ein weiterer Vorteil des erfindungsgemäßen Verfahrens besteht darin, daß anwesende anorganische Salze und organische Nebenprodukte die Reaktion nicht wesentlich stören. Dies ermöglicht es, daß das DAHNP, welches z.B. in bekannter Weise aus Natriumalkoholat, Guanidin-Salz und Cyanessigsäureester, dann Umsetzung des erhaltenen 2,4-Diamino-6-hydroxypyrimidins (DAHP) mit NaN0₂ und Säure gemäß Abb. 1 erhalten wird, 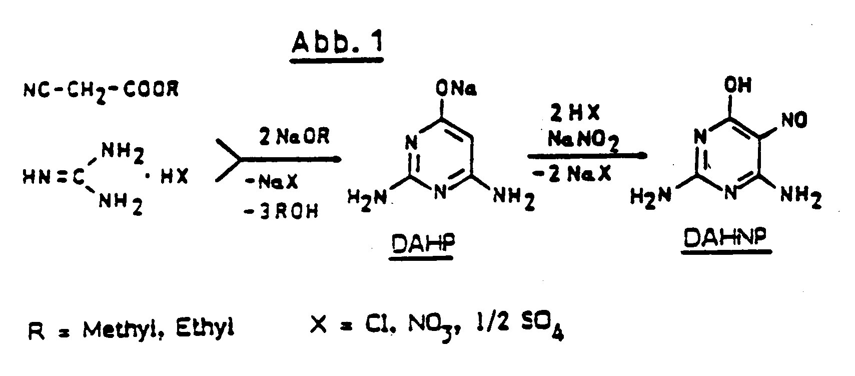
nicht in isolierter Form eingesetzt werden muß. Erfindungsgemäß kann die primär in der Synthese anfallende wäßrige Suspension, die pro mol DAHNP noch 2,6 bis 3,4 mol Natrium-Salze und geringe Mengen organischer Nebenprodukte enthält, eingesetzt werden. Auch das DAHP bzw. seine Natriumverbindung braucht nicht isoliert zu werden. Da das DAHNP nach den bekannten Herstellverfahren sehr fein und damit sehr schlecht filtrierbar anfällt, bringt die erfindungsgemäße Weiterverarbeitung als Suspension einen erheblichen Zeitgewinn.Allerdings ist es möglich, ohne die Erfindung und die Wirkung auf die Auflösbarkeit des Edelmetall-Katalysators zu beeinträchtigen, auch für eine kurze Zeit oder in kleiner Menge, alkalische Mittel zuzugeben und danach gegebenenfalls den pH-Wert wieder zu senken, soweit und solange dabei nicht der Edelmetall-Katalysator aufgelöst wird.

Die Beispiele erläutern besonders den Einfluß des pH auf das Verhalten der Edelmetalle. Die Ausbeute und Reinheit liegen gleich hoch oder besser als im Stand der Technik.

### Beispiel 1

In einem 2 l-Rührautoklaven wurde eine Suspension von 279,2 g (1,8 mol) DAHNP in 650 ml Wasser durch Zusatz von wenig Schwefelsäure auf pH 3,5 eingestellt, mit 5 g Gew.-%iger Pd-Kohle versetzt, dann auf 60 °C erwärmt und mit 15 bis 20 bar Wasserstoff beaufschlagt. Die Temperatur wurde auf 95 bis 100 °C gesteigert und nach Beendigung der Wasserstoffaufnahme noch 30 Minuten nachgerührt. Das Reaktionsgemisch wies nun einen pH von 5,5 bis 6,5 auf, wurde dann erst mit 1,98 mol wäßriger Natronlauge versetzt und dadurch auf pH 12 eingestellt. In bekannter Weise wurde danach vom Katalysator abfiltriert und in 3,15 mol wäßrige Schwefelsäure eingerührt. Nach Absaugen und Waschen wurden das erhaltene TAHP-Sulfat und das Abwasser mittels Atomabsorptionsspektrometrie (AAS) auf Pd untersucht. Innerhalb der Nachweisgrenze (1 ppm) wurde weder im Abwasser noch im TAHP-Sulfat Pd gefunden.

### Beispiel 2

Der nach Durchführung des Beispiels 1 zurückgewonnene Katalysator, der filterfeucht etwa 10 g wiegt, wurde zusammen mit 1 g frischen Katalysator in einem weiteren Ansatz, wie in Beispiel 1 beschrieben, eingesetzt. Auch hier wurde im Abwasser und im TAHP-Sulfat kein Pd gefunden. Der in diesem Ansatz zurückgewonnene Katalysator konnte jeweils unter Zusatz von 1 g frischem Katalysator problemlos in drei weiteren Ansätzen verwendet werden. Mittels AAS wurde auch hier kein Pd im Abwasser und im TAHP-Sulfat gefunden.

### Beispiel 3

Eine durch Zugabe von 0,4 mol Cyanessigsäuremethylester zu 0,8 mol Natriummethanolat in Methanol und 0,4 mol Guanidin-Nitrat in der Siedehitze, dann Abdestillation des Methanols und Zusatz von Wasser erhaltene Lösung des Natriumsalzes des DAHP wurde mit 0,4 mol Natriumnitrit versetzt und durch Einlaufenlassen in vorgelegte 0,44 mol wäßriger 30 Gew.-%iger Schwefelsäure in eine DAHNP-Suspension überführt. Am Beginn hatte die Suspension (Volumen 650 bis 700 ml) einen pH-Wert von 3,5. Es wurde mit 1,2 g 5 Gew.-%iger Pd-Kohle (entsprechend 60 mg Pd-Metall) versetzt und in einem 1 l-Hubautoklav bei 90 bis 100 °C und 50 bar Wasserstoffdruck hydriert. Nach Beendigung der Wasserstoffaufnahme wurde noch 60 Minuten nach-reagieren gelassen, der pH der Suspension betrug dann 6 bis 6,5. Dann wurde mit 0,44 mol wäßriger Natronlauge versetzt und entsprechend Beispiel 1 vom Katalysator abfiltriert und in 0,7 mol wäßrige Schwefelsäure eingerührt, das erhaltene TAHP-Sulfat abfiltriert und gewaschen. Mittels AAS wurde auch hier kein Pd im TAHP-Sulfat und im Abwasser nachgewiesen.

### Beispiel 4

Es wurde entsprechend Beispiel 3 verfahren. Vor Reaktionsbeginn hatte die Suspension einen pH-Wert von 8,5. Unmittelbar nach der Reaktion betrug der pH dann 7,5 bis 9. Mittels AAS wurde auch hier kein Pd im TAHP-Sulfat und im Abwasser nachgewiesen.

### Beispiel 5

Eine entsprechend Beispiel 3 aus 8,0 mol Cyanessigsäuremethylester, 16,0 mol Natriummethanolat, 8,0 mol Guanidin-Nitrat, 8,0 mol Natriumnitrit und 8,8 mol Schwefelsäure erhaltene DAHNP-Suspension hatte einen pH-Wert von 3,5. Das Volumen betrug 13 l. Dann wurde mit 30 bar und 25 g 5 Gew.-%iger Pd-Kohle bei 70 bis 80 °C in einem V₄A-Treibstrahl-Schlaufenreaktor hydriert. Nach Beendigung der Wasserstoffaufnahme wurde noch 30 Min. nachreagieren gelassen, der pH betrug dann 6 bis 7. Mit 8,8 mol Natronlauge und 14,0 mol Schwefelsäure wurde entsprechend Beispiel 2 aufgearbeitet. Mittels AAS wurde auch hier im TAHP-Sulfat und im Abwasser kein Pd gefunden.

### Beispiel 6

Es wurde entsprechend Beispiel 3 verfahren, jedoch betrug vor Reaktionsbeginn der pH-Wert 2,0. Statt 1,2 g 5 Gew.-%iger Pd-Kohle wurden 2,5 g 5 Gew.-%iger Pt-Kohle eingesetzt. Unmittelbar nach der Reaktion betrug der pH 6,0. Mittels AAS wurde kein Pt im TAHP-Sulfat und im Abwasser gefunden.

### Vergleichsbeispiel A

Es wurde entsprechend Beispiel 3 verfahren, jedoch vor Reaktionsbeginn mit verdünnter Natronlauge ein pH von 11,0 eingestellt. Unmittelbar nach der Reaktion betrug der pH dann 10,2. Mittels AAS wurden im TAHP-Sulfat und im Abwasser insgesamt ca. 20 bis 30 mg Pd gefunden, das sind 33 bis 50 % des eingesetzten Edelmetalls.

### Vergleichsbeispiel B

Es wurde entsprechend Beispiel 3 verfahren, jedoch vor Reaktionsbeginn ein pH von 11,3 eingestellt und statt 1,2 g 5 Gew.- %iger Pd-Kohle wurden 2,5 g 5 Gew.-%iger Pt-Kohle eingesetzt. Unmittelbar nach der Reaktion betrug der pH 10. Mittels AAS wurden im Abwasser 55 mg Pt nachgewiesen, das sind 44 % des eingesetzten Pt-Metalls.

## Patentansprüche

1. Verfahren zur Herstellung von 2,4,5-Triamino-6-hydroxypyrimidin (TAHP) durch katalytische Hydrierung von 2,4-Diamino-6-hydroxy-5-nitrosopyrimidin (DAHNP) bei erhöhtem Druck und erhöhter Temperatur in einem wäßrigen, gegebenenfalls noch organische Lösemittel und/oder inerte Salze enthaltenden Medium, unter Verwendung eines Edelmetall-Hydrierkatalysators, *dadurch gekennzeichnet,* daß die Hydrierung im sauren, neutralen oder schwach alkalischen Bereich unterhalb pH 9 erfolgt, und wobei vorzugsweise vor und während der Hydrierung die Zugabe alkalischer Mittel unterbleibt und nach der Hydrierung die zur Lösung von TAHP notwendige Menge alkalischer Mittel zugegeben und die entstehende Lösung vom Katalysator getrennt wird.

2. Verfahren nach Anspruch 1*, dadurch gekennzeichnet,* daß der pH-Wert während der Hydrierung unter 9,0, vorzugsweise zwischen 3 und 8,5 liegt.

3. Verfahren nach Anspruch 1 oder 2, *dadurch gekennzeichnet,* daß als Base zum Lösen des gebildeten TAHP nach beendeter Reaktion 0,9 bis 1,2 mol Natrium- oder Kaliumhydroxyd pro Mol des eingesetzten DAHNP verwendet wird.

4. Verfahren nach Anspruch 1 bis 3, *dadurch gekennzeichnet,* daß die Konzentration an DAHNP 0,4 bis 3,0 mol/l beträgt.

5. Verfahren nach einem der Anspruche 1 bis 4, *dadurch gekennzeichnet,* daß die Hydrierung bei einem Wasserstoffdruck von 3 bis 150 bar, vorzugsweise 10 bis 60 bar, durchgeführt wird.

6. Verfahren nach einem der Ansprüche 1 bis 5, *dadurch gekennzeichnet,* daß als Hydrierkatalysatoren Ru, Pd, Pt oder deren Verbindungen, vorzugsweise Pd, PdO, Pt, PtO₂ auf Kohle als Trägermaterial, eingesetzt werden.

7. Verfahren nach einem der Ansprüche 1 bis 6, *dadurch gekennzeichnet,* daß die Hydrierung bei Temperaturen von 50 bis 150 °C, vorzugsweise 70 bis 120 °C, durchgeführt wird.

8. Verfahren nach einem der Ansprüche 1 bis 7, *dadurch gekennzeichnet,* daß DAHNP in Form einer wäßrigen Suspension eingesetzt wird, die bei der Herstellung dieser Verbindung in an sich bekannter Weise ausgehend von Guanidin-Salzen, Alkalialkoholaten und Cyanessigsäureestern und nachfolgender Nitrosierung ohne Isolierung von Zwischenstufen und ohne Abtrennung von Nebenprodukten erhalten wird.

9. Verfahren nach einem der Ansprüche 1 bis 8, *dadurch gekennzeichnet,* daß der gebrauchte Katalysator in den folgenden Ansätzen jeweils unter Zusatz von 0 bis 50 % frischem Katalysator wieder eingesetzt wird.

## Claims

1. Process for the preparation of 2,4,5-triamino-6-hydroxypyrimidine (TAHP) by catalytic hydrogenation of 2,4-diamino-6-hydroxy-5-nitrosopyrimidine (DAHNP) at elevated pressure and elevated temperature in an aqueous medium optionally containing in addition, organic solvent and/or inert salts, with use of a noble metal hydrogenation catalyst, **characterised in that** the hydrogenation takes place in the acid, neutral or weakly alkaline range below pH 9 and with the addition of alkaline agent preferably not taking place before and during the hydrogenation and, after the hydrogenation, the necessary amount of alkaline agent being added to the solution of TAHP, and the solution obtained being separated from catalyst.

2. Process according to claim 1, **characterised in that** the pH value during the hydrogenation is under 9.0, preferably between 3 and 8.5.

3. Process according to claim 1 or 2, **characterised in that**, after the end of the reaction, 0.9 to 1.2 mol of sodium or potassium hydroxide are used per mol of the DAHNP employed as base for the dissolution of the TAHP formed.

4. Process according to claim 1 to 3, **characterised in that** the concentration of DAHNP amounts to 0.4 to 3.0 mol/l.

5. Process according to one of claims 1 to 4, **characterised in that** the hydrogenation is carried out at a hydrogen pressure of 3 to 150 bar, preferably 10 to 60 bar.

6. Process according to one of claims 1 to 5, **characterised in that** Ru, Pd, Pt or compounds thereof, preferably Pd, PdO, Pt, PtO₂ on charcoal as support material, are employed as hydrogenation catalysts.

7. Process according to claims 1 to 6, **characterised in that** the hydrogenation is carried out at temperatures of 50 to 150°C, preferably 70 to 120°C.

8. Process according to one of claims 1 to 7, **characterised in that** DAHNP is employed in the form of an aqueous suspension which is obtained, in known manner, starting from guanidine salts, alkali alcoholates and cyanoacetic acid esters and a subsequent nitrosation without isolation of intermediate steps and without separation off of by-products.

9. Process according to one of claims 1 to 8, **characterised in that** the catalyst used is employed again in subsequent batches in each case with addition of 0 to 50% by weight of fresh catalyst.

## Revendications

1. Procédé de préparation de la 2,4,5-triamino-6-hydroxypyrimidine (TAHP) par hydrogénation catalytique de la 2,4-diamino-6-hydroxy-5-nitrosopyrimidine (DAHNP), sous haute pression et à haute température, dans un milieu aqueux qui contient encore éventuellement des solvants organiques et/ou des sels inertes, sous l'action d'un catalyseur d'hydrogénation à base de métal noble, lequel procédé est caractérisé en ce que l'hydrogénation est effectuée dans un milieu acide, neutre ou faiblement alcalin dont le pH est inférieur à 9, et dans lequel procédé on n'ajoute de préférence pas d'agent alcalin ni avant, ni pendant l'hydrogénation, mais on ajoute, après l'hydrogénation, la quantité d'agent alcalin nécessaire pour dissoudre la TAHP et on sépare le catalyseur d'avec la solution formée.

2. Procédé conforme à la revendication 1, caractérisé en ce que, pendant l'hydrogénation, le pH est inférieur à 9,0 et vaut de préférence entre 3 et 8,5.

3. Procédé conforme à la revendication 1 ou 2, caractérisé en ce que, pour dissoudre la TAHP formée après la fin de la réaction, on utilise, comme base, de 0,9 à 1,2 mole d'hydroxyde de sodium ou de potassium par mole de DAHNP mise en jeu dans la réaction.

4. Procédé conforme à l'une des revendications 1 à 3, caractérisé en ce que la concentration de DAHNP vaut de 0,4 à 3,0 mol/l.

5. Procédé conforme à l'une des revendications 1 à 4, caractérisé en ce que l'on effectue l'hydrogénation sous une pression d'hydrogène valant de 3 à 150 bar et de préférence de 10 à 60 bar.

6. Procédé conforme à l'une des revendications 1 à 5, caractérisé en ce que l'on utilise, comme catalyseur d'hydrogénation, du ruthénium, du palladium, du platine ou un de leurs composés, de préférence Pd, PdO, Pt ou PtO₂, supporté sur du charbon.

7. Procédé conforme à l'une des revendications 1 à 6, caractérisé en ce que l'on effectue l'hydrogénation à une température valant de 50°C à 150°C, et de préférence de 70°C à 120°C.

8. Procédé conforme à l'une des revendications 1 à 7, caractérisé en ce que l'on utilise la DAHNP sous la forme d'une suspension aqueuse obtenue lorsqu'on prépare ce composé, de façon connue, à partir d'un sel de guanidine, d'un alcoolate de métal alcalin et d'un ester de l'acide cyanoacétique, avec nitrosation ultérieure, sans isoler le produit intermédiaire et sans séparer les sous-produits.

9. Procédé conforme à l'une des revendications 1 à 8, caractérisé en ce que l'on réutilise pour les charges suivantes le catalyseur usé, en y ajoutant à chaque fois de 0 à 50 % de catalyseur neuf.
